# EUROPEAN PATENT APPLICATION

(11) **EP 4 574 141 A1**
(43) Date of publication of application: **25.06.2025**
(21) Application number: 24194560.9
(22) Date of filing: 14.08.2024
(51) Int. Cl.: A61K 9/51, A61K 39/00, A61K 47/69, C12N 15/88

(54) **LIPID PARTICLE, COMPOSITION CONTAINING LIPID PARTICLE, KIT CONTAINING LIPID PARTICLE, AND ACTIVE AGENT DELIVERY METHOD USING LIPID PARTICLE**

(30) Priority: 19.12.2023 JP 2023214079
(71) Applicant: Kabushiki Kaisha Toshiba, Minato-ku Tokyo (JP)
(72) Inventor: TAGE, Hiroki, Tokyo (JP); AKAHOSHI, Eiichi, Tokyo (JP); ISHIHARA, Mitsuko, Tokyo (JP)
(74) Representative: Marks & Clerk LLP

(57) **Abstract**

Provided are a lipid particle with high biodegradability, high biocompatibility, a high active agent introducibility, low cytotoxicity, and the like, a composition and a kit which use the lipid particle, and a method of delivering an active agent to a cell.

The lipid particle according to the present embodiment includes:
a compound represented by the following Formula (a),
Mal-L^{a1}-(OCH₂CH₂)ₙₐ-O-L^{a2}R^{a}₂ (a)
wherein, in Formula (a),
Mal is a maleimide group,
L^{a1} is an unsubstituted hydrocarbon group, or a substituted hydrocarbon group,
L^{a2} is a trivalent hydrocarbon group that does not contain phosphorus,
each R^{a} is a hydrocarbon group containing at least one ester bond at a predetermined position, and
na is a number of 1 or more; and
an active agent. Also, a composition and a kit according to an embodiment include the lipid particle. Further, a method of delivering an active agent to a cell according to an embodiment includes bringing the lipid particle with a cell.

## Description

### TECHNICAL FIELD

The present invention relates to a lipid particle containing a biodegradable compound having a structure that is degraded in a cell. The invention also relates to a composition and a kit which include the lipid particle and are used for delivery of an active agent such as a nucleic acid.

### BACKGROUND ART

Liposomes for the purpose of treating various diseases have been studied. Liposomes are minute capsules composed of lipids and having a particle size on the order of nanometers, can contain various compounds and the like therein, and are excellent in biocompatibility and the like, and thus are ideal materials for selectively delivering therapeutic agents or active agents to a target site in a living body. For such a purpose, various materials have been developed as materials constituting the liposomes.

Such liposomes can be composed of a single lipid. In such a case, for example, a phospholipid having a head portion and a hydrophobic portion bound to the head portion is used for the lipid, and the lipid associates to form a membrane to constitute minute capsules capable of encapsulating an active agent or the like. However, in order to impart excellent properties to liposomes, lipid mixtures are generally used to constitute the capsules. The lipid mixtures contain a combination of a lipid having excellent biodegradability, a lipid suppressing aggregation of liposomes to be formed, a lipid having an effect of suppressing leakage of the inclusion, a lipid having a membrane fusion effect, and the like.

In order to further improve the properties of the liposomes, individual lipids have been studied. For example, it is preferable that medical liposomes specialized for gene transfer have high biodegradability, high biocompatibility, high active agent introducibility, and low cytotoxicity. As such liposomes, liposomes whose surfaces are modified with a maleimide group are known (Patent Literature 1).

Such liposomes exhibit excellent properties, but it is desirable to increase the number of kinds of lipids that can be selected according to the state of a living body which they are applied, a disease to be treated, and the like. Furthermore, there is a need for lipid particles capable of constituting liposomes having properties superior to those of conventional liposomes.

### CITATION LIST

### PATENT LITERATURE

Patent Literature 1: JP 2006-241107 A

### SUMMARY OF THE INVENTION

### OBJECT OF THE INVENTION

In view of the above objects, the present embodiment provides a lipid particle containing a specific biodegradable compound and an active agent, a composition and a kit which use the lipid particle, and an active agent delivery method using the lipid particle.

### SOLUTION TO PROBLEM

According to the present invention, the following invention is provided.
[1] A lipid particle comprising:
   a compound represented by the following Formula (a),

      Mal-L^{al}-(OCH₂CH₂)ₙₐ-O-L^{a2}R^{a}₂ (a)
   wherein, in Formula (a),
   Mal is a maleimide group,
   L^{a1} is an unsubstituted hydrocarbon group, or a substituted hydrocarbon group containing a linking group selected from the group consisting of -C(=O)-O-, -O-C(=O)-, -O-C(=O)-O-, -S-C(=O)-, -C(=O)-S-, -C(=O)-NH-, and -NH-C(=O)-,
   L^{a2} is a trivalent hydrocarbon group that does not contain phosphorus,
   each R^{a} is independently a C₁₀-C₃₀ hydrocarbon group containing at least one ester bond at a predetermined position, and
   na is a number of 1 or more; and
   an active agent.
[2] The lipid particle according to [1], in which na is a number from 10 to 100.
[3] The lipid particle according to [1] or [2], in which the compound represented by Formula (a) is dimyristoyl glycerol polyethylene glycol maleimide.
[4] The lipid particle according to any one of [1] to [3], further comprising a compound represented by the following Formula (b),

   Q-CHR^{b}₂ (b)

   wherein, in Formula (b),
   Q is a nitrogen-containing aliphatic group that contains two or more tertiary nitrogen atoms and does not contain oxygen,
   each R^{b} is independently a C₁₂-C₂₄ aliphatic group, and at least one R^{b} contains a linking group selected from the group consisting of -C(=O)-O-, -O-C(=O)-, -O-C(=O)-O-, -S-C(=O)-, -C(=O)-S-, -C(=O)-NH-, and - NH-C(=O)- in a main chain or a side chain thereof).
[5] The lipid particle according to [4],
   in which Q is represented by the following Formula (Q'):

      R^{Q1}₂N-(CR^{Q2}₂)_{q1}-NR^{Q1}-(CR^{Q2}₂)_{q2}-*2 (Q')
   wherein, in Formula (Q'),
   each R^{Q1} is independently an alkyl group,
   each R^{Q2} is independently hydrogen or alkyl, or
   any two of the R^{Q1} and R^{Q2} groups may form one alkylene group to form a nitrogen-containing alicyclic ring,
   q1 is a number of 1 to 5,
   q2 is a number of 0 to 5, and
   *2 represents a bonding position to -CHR^{b}₂).
[6] The lipid particle according to any one of [1] to [3], further comprising an additional lipid compound selected from the group consisting of a cationic lipid, a neutral lipid, a structure-forming lipid, and an aggregation-reducing lipid.
[7] The lipid particle according to any one of [1] to [6], in which the active agent is a nucleic acid selected from the group consisting of a plasmid, an oligonucleotide, a polynucleotide, siRNA, microRNA, DNA, mRNA, an aptamer, and a ribozyme.
[8] The lipid particle according to [7], further comprising a compound that binds to a nucleic acid.
[9] The lipid particle according to [8], in which the compound that binds to the nucleic acid is a basic protein or a basic peptide.
[10] The lipid particle according to any one of [1] to [9], further comprising a compound that modulates expression of a nucleic acid in a cell.
[11] A composition comprising: the lipid particle according to any one of [1] to [10]; and a carrier.
[12] A composition for delivering an active agent to a cell, comprising: the lipid particle according to any one of [1] to [10]; and a carrier.
[13] The composition according to [12], in which the cell is a mammalian cell.
[14] The composition according to [13], in which the mammalian cell is a hematopoietic cell.
[15] The composition according to [14], in which the hematopoietic cell is selected from the group consisting of a PBMC, a T cell, a dendritic cell, and a CD34 positive cell.
[16] A method of delivering an active agent to a cell, comprising bringing the lipid particle according to any one of [1] to [10] into contact with a cell.
[17] The method of delivering an active agent according to [16], in which the cell is a cell of an animal other than a human or a cell extracted from a living body.
[18] The method of delivering an active agent according to [16] or [17], in which the lipid particle further comprises a lipid that forms a membrane and a lipid capable of reducing aggregation.
[19] The method of delivering an active agent according to [18],
   in which the lipid that forms a membrane is selected from the group consisting of:
   1,2-dioleoyl-sn-glycero-3-phosphoethanolamine (DOPE),
   1,2-distearoyl-sn-glycero-3-phosphoethanolamine (DSPE),
   1,2-dipalmitoyl-sn-glycero-3-phosphatidylcholine (DPPC),
   1-palmitoyl-2-oleoyl-sn-glycero-3-phosphatidylcholine (POPC),
   1,2-di-O-octadecyl-3-trimethylammonium propane (DOTMA),
   1,2-dioleoyl-3-dimethylammonium propane (DODAP),
   1,2 dimyristoyl-3-dimethylammonium propane (14:0 DAP),
   1,2 dipalmitoyl-3-dimethylammonium propane (16:0 DAP),
   1,2 distearoyl-3-dimethylammonium propane (18:0 DAP),
   N-(4-carboxybenzyl)-N,N-dimethyl-2,3-bis(oleoyloxy)propane (DOBAQ),
   1,2-dioleoyl-3-trimethylammonium propane (DOTAP),
   1,2 dioleoyl-sn-glycero-3-phosphocholine (DOPC),
   1,2-dilinoleoyl-sn-glycero-3-phosphocholine (DLPC),
   1,2-dioleoyl-sn-glycero-3-phospho-L-serine (DOPS), and
   cholesterol, and
   the lipid capable of reducing aggregation is a polyethylene glycol (PEG) modified lipid.
[20] The method of delivering an active agent according to any one of [16] to [19], in which the active agent is a nucleic acid selected from the group consisting of a plasmid, an oligonucleotide, a polynucleotide, an siRNA, microRNA, DNA, mRNA, an aptamer, and a ribozyme.
[21] The method of delivering an active agent according to any one of [16] to [19], in which the active agent comprises a combination of at least one type of DNA and at least one type of RNA.
[22] The method of delivering an active agent according to [20] or [21], in which the lipid particle further comprises a compound that binds to a nucleic acid.
[23] The method of delivering an active agent according to [22], in which the compound that binds to a nucleic acid is a basic protein or a basic peptide.
[24] The method of delivering an active agent according to [22], in which the compound that binds to a nucleic acid is protamine or a histone.
[25] The method of delivering an active agent according to [22], in which the lipid particle further comprises a compound that modulates expression of a nucleic acid in a cell.
[26] The method of delivering an active agent according to any one of [16] to [25], further comprising bringing a carrier into contact with the cell, along with the lipid particle.
[27] The method of delivering an active agent according to any one of [16] to [26], in which the cell is a tumor cell.
[28] A method of preparing a cell comprising:
   performing the method of delivering an active agent according to any one of [16] to [27]; and
   culturing the obtained cell.
[29] A kit comprising: the lipid particle according to any one of [1] to [10]; and an introduction agent that introduces the lipid particle into a cell.
[30] The kit according to [29], further comprising a substance that improves storage stability of the lipid particle.

### BRIEF DESCRIPTION OF DRAWINGS

Fig. 1 is a graph showing an effect of introducing an active agent into human peripheral blood mononuclear cells (PBMC) according to Examples and Comparative Examples.
Fig. 2 is a graph showing an effect of introducing an active agent into human T-cell leukemia cells (Jurkat) according to Examples and Comparative Examples.
Fig. 3 is a graph showing an effect of introducing an active agent into human peripheral blood mononuclear cells (PBMC) according to Examples and Comparative Examples.
Fig. 4 is a graph showing an effect of introducing an active agent into human mammary adenocarcinoma cells (MDA-MB231) according to Examples and Comparative Examples.
Fig. 5 is a graph showing the effect of introducing an active agent into human T-cell leukemia cells (Jurkat) according to Examples and Comparative Examples.
Fig. 6 is a graph showing an effect of introducing an active agent into human mammary adenocarcinoma cells (MDA-MB231) according to Examples and Comparative Examples.
Fig. 7 is a graph showing an effect of introducing an active agent into human T-cell leukemia cells (Jurkat) according to Examples and Comparative Examples.

### [Definitions]

In the present embodiment, in a case where a numerical range is indicated using "to", unless otherwise specified, these numerical ranges include both end points, and have common units. For example, 10 to 25 mol% means 10 mol% or more and 25 mol% or less.

In the present embodiment, the expression such as "Cₓ to C_{y}" and "Cₓ" means the number of carbon atoms in a molecule or a substituent. For example, C₁-C₆ alkyl means alkyl having 1 or more and 6 or less carbon atoms. In the present embodiment, an alkyl halide refers to one in which one or more hydrogen atoms in an alkyl group are substituted with a halogen such as fluorine, and for example, a fluoroaryl group refers to one in which one or more hydrogen atoms in an aryl group are substituted with fluorine.

In the present embodiment, unless otherwise specified, alkyl means a monovalent group obtained by removing one hydrogen from any carbon of an alkane. The term alkyl includes linear or branched alkyl. Furthermore, cycloalkyl means alkyl having a cyclic structure. A cyclic structure substituted with a linear or branched alkyl is also referred to as cycloalkyl.

In addition, an alkenyl means a monovalent group obtained by removing one hydrogen atom from any carbon atom of an alkene.

The unsubstituted hydrocarbon group means a monovalent or divalent or higher valent group containing carbon and hydrogen. The substituted hydrocarbon group means a monovalent or divalent or higher valent group containing oxygen, nitrogen, or sulfur in addition to carbon and hydrogen. The substituted hydrocarbon group may optionally contain a heteroelement such as a halogen. When simply referred to as a hydrocarbon group, the term includes both an unsubstituted hydrocarbon group and a substituted hydrocarbon group. The aliphatic group is a hydrocarbon group that does not contain an aromatic ring, and may have any of a chain structure, a branched chain structure, and a cyclic structure, or may have a combination thereof. In addition, unless otherwise specified, the aliphatic group may contain an unsaturated bond. Furthermore, unless otherwise specified, the aliphatic group may contain a heteroatom such as nitrogen, oxygen, sulfur, selenium, fluorine, chlorine, or bromine. The aliphatic group may be a monovalent group or a multivalent group. The aromatic hydrocarbon group includes an aromatic ring, and has an aliphatic hydrocarbon group as a substituent as necessary.

The tertiary nitrogen means a nitrogen to which three carbons are bonded. Therefore, the tertiary nitrogen forms a tertiary amine structure having an electron donating property.

### [Lipid Compound]

A compound according to an embodiment is a compound suitable as a lipid constituting a liposome, having a maleimide group and a polyethylene glycol chain. This compound is represented by the following Formula (a).

Mal-L^{al}-(OCH₂CH₂)ₙₐ-O-L^{a2}R^{a}₂ (a)

wherein, in Formula (a),
Mal is a maleimide group,
L^{a1} is an unsubstituted hydrocarbon group, or a substituted hydrocarbon group containing a linking group selected from the group consisting of -C(=O)-O-, -O-C(=O)-, -O-C(=O)-O-, -S-C(=O)-, -C(=O)-S-, -C(=O)-NH-, and -NH-C(=O)-,
L^{a2} is a trivalent hydrocarbon group that does not contain phosphorus,
each R^{a} is independently a C₁₀-C₃₀ hydrocarbon group containing at least one ester bond at a predetermined position, and
na is a number of 1 or more.

The maleimide group Mal has a structure represented by the following Formula (m). 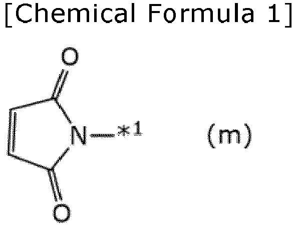 (In Formula (m), *1 represents the bonding position to -L^{a1}-)

L^{a1} is an unsubstituted hydrocarbon group, or a substituted hydrocarbon group containing a linking group selected from the group consisting of -C(=O)-O-, -O-C(=O)-, -O-C(=O)-O-, -S-C(=O)-, -C(=O)-S-, -C(=O)-NH-, and -NH-C(=O)-.

In a case where L^{a1} is a substituted hydrocarbon group, it is preferable that L^{a1} be bonded to a hydrocarbon chain without interposing a linking group with respect to a maleimide group. The number of carbon atoms of the unsubstituted hydrocarbon group and the number of carbon atoms in the hydrocarbon chain included in the substituted hydrocarbon group are preferably 1 to 5 and more preferably 2 or 3. L^{a1} of the unsubstituted hydrocarbon group is most preferably -(CH₂)₂-. The linking group in the substituted hydrocarbon group may include oxygen or nitrogen. -C(=O)-NH- is particularly preferable. L^{a1} containing a substituted carbon group is most preferably -(CH₂)₂-C(=O)-NH-.

-(OCH₂CH₂)ₙₐ-O- is a linear polyethylene glycol chain. The polyethylene glycol chain improves hydrophilicity of a compound, and when a liposome is constituted by the compound, cytotoxicity to cells tends to decrease, and an introduction rate of an active agent such as a nucleic acid tends to increase.

na is the number indicating the degree of polymerization of an ethyleneoxy group, is 1 or more, is preferably 20 to 70, and more preferably 30 to 60.

L^{a2} is a trivalent linking group that links a polyethylene glycol chain and two R^{a}'s. It is necessary that L^{a2} do not contain phosphorus. According to the study of the present inventors, it was found that when L^{a2} contains phosphorus, an introduction rate of an active agent tends to be low. Therefore, L^{a2} is typically preferably composed only of carbon and hydrogen, and may contain oxygen as necessary. In addition, in an embodiment, it is preferable that the compound represented by Formula (a) do not contain phosphorus at all in the structure.

The hydrocarbon group L^{a2} generally has a structure in which three hydrogens are excluded from the hydrocarbon. If necessary, at least one of methylene -CH₂- contained in the hydrocarbon may be substituted with a divalent group containing oxygen, for example, a carboxylate group - C(=O)-O-, an oxy group -O-, or a carbonyl group -C(=O)-.

R^{a}'s form a hydrophobic part of the compound represented by Formula (a), and two R^{a}'s may be the same or different. R^{a}'s are each independently a C₁₀-C₃₀ hydrocarbon group containing at least one ester bond at a predetermined position. This ester group functions as a biodegradable group in a case where a compound according to an embodiment is used in a liposome.

A preferred example of R^{a} can be represented by the following Formula (ra).

-L^{ra1}-C( = O)-O-L^{ra2} (ra)

wherein in Formula (ra),
L^{ra1} is alkylene, and
L^{ra2} is a hydrocarbon group)

L^{ra1} and L^{ra2} may have a branched chain structure or a cyclic structure, and in a case of having a branched structure, it is preferable that the number of side chains be small, and it is most preferable that it have a linear structure. L^{ra2} may be a saturated hydrocarbon group or an unsaturated hydrocarbon group.

Each site of the compound represented by Formula (a) used in the lipid particle according to an embodiment has the structure as described above, and the compound according to an embodiment preferably has structures represented by the following Formulas (a-01) to (a-12).

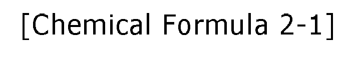

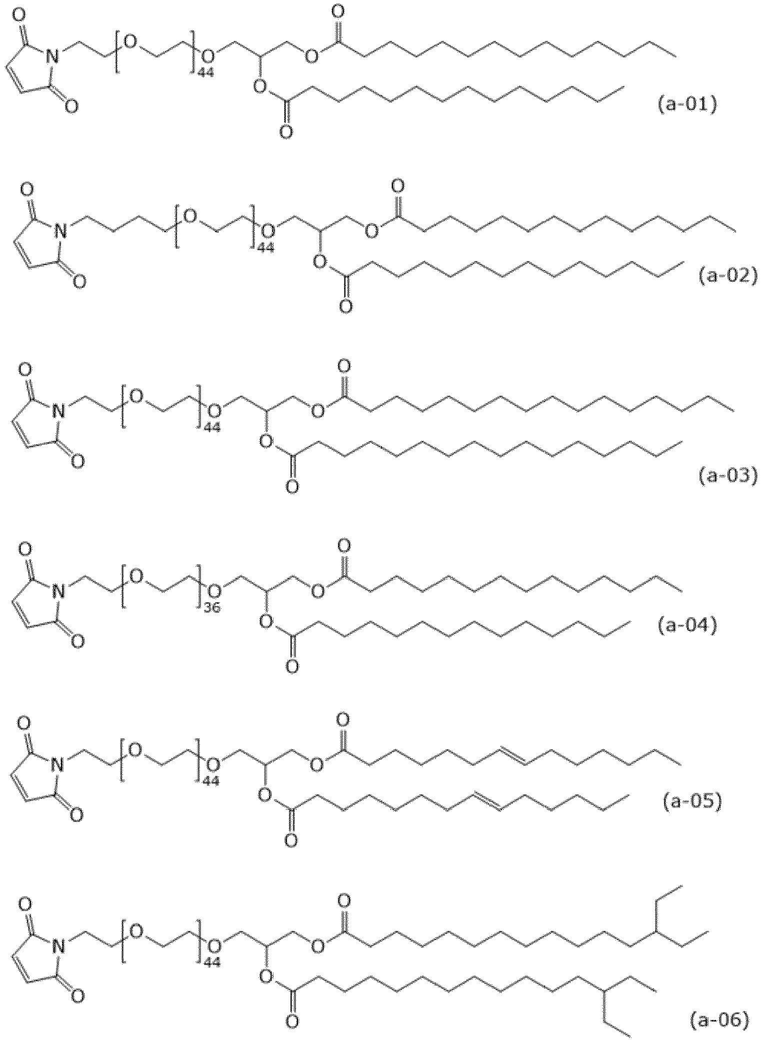

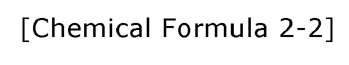

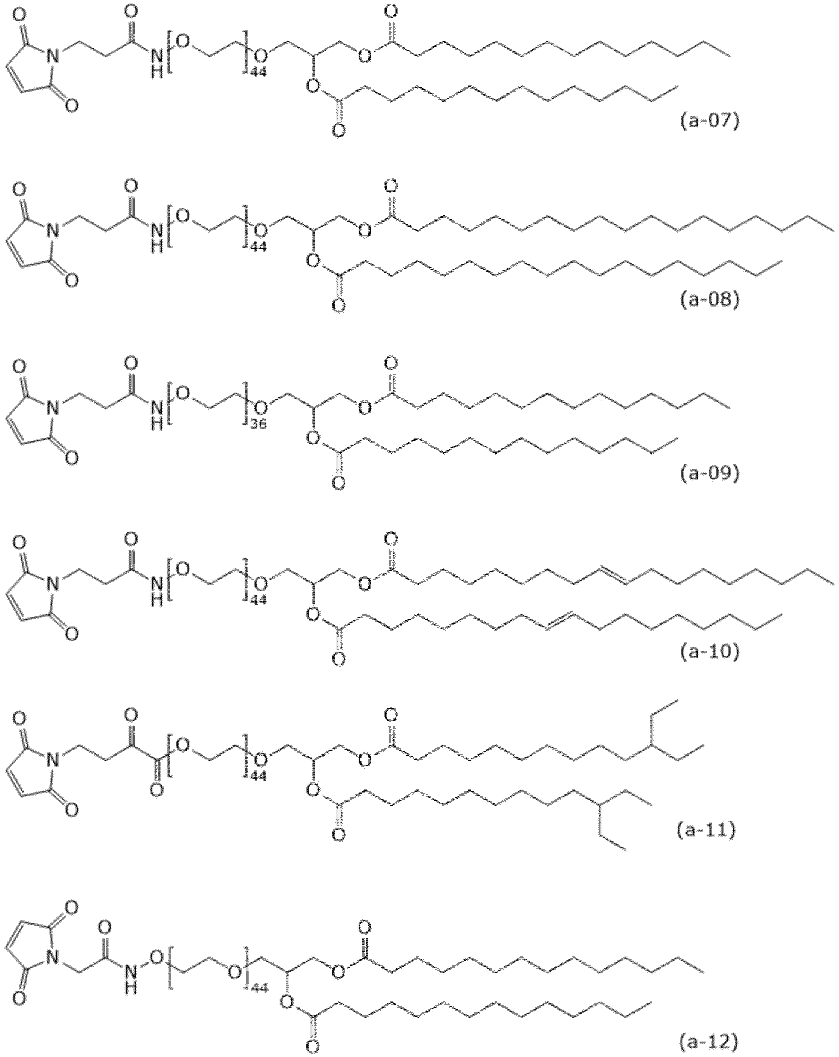

Among the compounds represented by Formula (a), dimyristoyl glycerol polyethylene glycol maleimide represented by Formula (a-01) is most preferable.

### [Additional Lipid Compound]

The lipid particle according to an embodiment comprises the compound represented by Formula (a) as a lipid compound, and preferably further comprises an additional lipid compound. Such an additional lipid compound can be arbitrarily selected from those generally used for lipid particles.

For example, the lipid particle according to an embodiment preferably further comprises a compound represented by the following Formula (b) as an additional lipid compound.

Q-CHR^{b}₂ (b)

wherein, in Formula (b),
Q is a nitrogen-containing aliphatic group that contains two or more tertiary nitrogen atoms and does not contain oxygen,
each R^{b} is independently a C₁₂-C₂₄ aliphatic group, and at least one R^{b} contains a linking group selected from the group consisting of -C(=O)-O-, -O-C(=O)-, -O-C(=O)-O-, -S-C(=O)-, -C(=O)-S-, -C(=O)-NH-, and - NH-C(=O)- in a main chain or a side chain thereof.

One feature of the compound represented by Formula (b) is that a head Q contains two or more tertiary nitrogen atoms and does not contain oxygen.

The head Q may further contain nitrogen constituting unsubstituted amino, quaternary ammonium, or the like as long as an effect of an embodiment is not impaired, and it is preferable that nitrogen other than the tertiary nitrogen be not contained. The head Q does not contain oxygen. Therefore, the head Q does not have a structure such as oxy, hydroxy, carboxy, alkoxy, or carboxylate. The head Q can contain an ionic group, and is preferably a neutral group that does not contain a polar group.

A preferred example of Q can be represented by the following General Formula (Q').

R^{Q1}₂N-(CR^{Q2}₂)_{q1}-NR^{Q1}-(CR^{Q2}₂)_{q2}-*2 (Q')

wherein, in Formula (Q'),
each R^{Q1} is independently an alkyl group,
each R^{Q2} is independently hydrogen or alkyl,
any two of the R^{Q1} and R^{Q2} groups may be bonded to form a nitrogen-containing alicyclic ring,
q1 is a number of 1 to 5, and preferably 1 to 4,
q2 is a number of 0 to 5, and preferably 1 to 4, and
*2 represents a binding site to -CHR^{b}₂.

Here, the alkyl is preferably a C₁-C₃ alkyl.

Any two of the R^{Q1} and R^{Q2} groups can be bonded to form a nitrogen-containing alicyclic ring. The number of constituent members of the nitrogen-containing alicyclic ring is not particularly limited, and is preferably 4 to 10 and more preferably 5 to 8. Typically, examples of the nitrogen-containing alicyclic ring to be constituted include piperidine, piperazine, pyrrolidine, imidazolidine, hexamethyleneimine, homopiperazine, and heptamethyleneimine.

Such Q has, for example, the following structure. 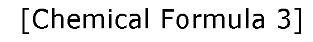 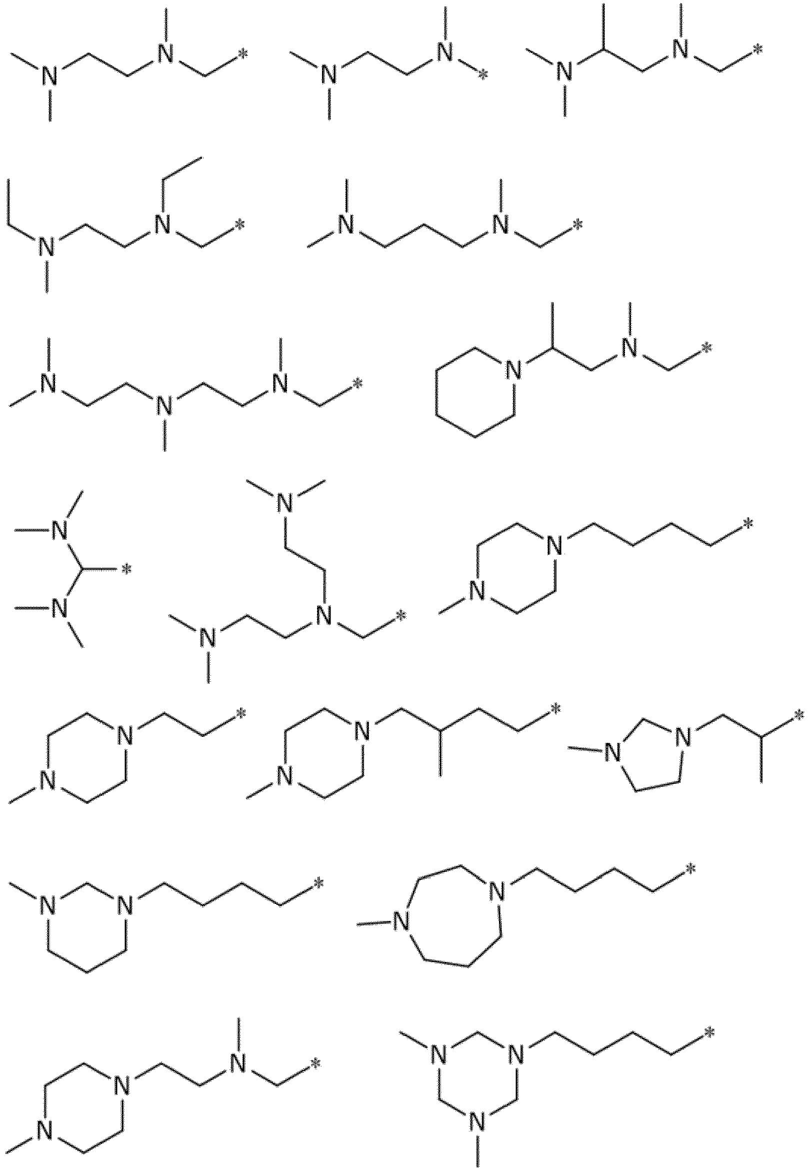 (In Chemical Formula 3, * represents a bonding position to -CHR^{b}₂)

The compound represented by Formula (b) has -CHR^{b}₂ bonded to the head. Here, the R^{b} groups are a hydrophobic group, and two of the R^{b} groups may be the same as or different from each other. The hydrophobic group generally has a relatively long hydrocarbon chain. Further, a part thereof has a linking group containing carboxylate or the like, specifically, a linking group selected from the group consisting of -C(=O)-O-, -O-C(=O)-, -O-C(=O)-O-, -S-C(=O)-, -C(=O)-S-, -C(=O)-NH-, and -NH-C(=O)-. These linking groups function as biodegradable groups in a case where a compound according to an embodiment is used in a liposome.

A preferred example of R^{b} can be represented by the following Formula (rb).

-L^{rb1}-C( = O)-O-L^{rb2} (rb)

wherein, in Formula (rb),
L^{rb1} is alkylene, and
L^{rb2} is alkenyl.

L^{rb1} and L^{rb2} may have a branched chain structure or a cyclic structure, and in a case of having a branched structure, it is preferable that the number of side chains be small, and it is most preferable that it have a linear structure.

More specifically, L^{rb1} and L^{rb} are preferably represented by the following Formulas (rb-1) and (rb-2), respectively.

-(CH₂)_{rb1}- (rb-1)

-CH₂-CH=CH-(CH₂)_{rb2}-H (rb-2)

wherein, in Formulae (rb-1) and (rb-2),
rb1 is a number of 1 to 10, and
rb2 is a number of 1 to 10.

Here, in order for R^{b} to exhibit sufficient hydrophobicity, rb1 is preferably a number of 4 to 8, and the longest molecular chain contained in R^{b} has preferably 8 atoms or more.

Each site of a compound according to an embodiment has the structure as described above, and the compound according to an embodiment preferably has structures represented by the following Formulas (b-01) to (b-21).

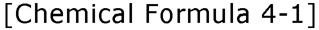

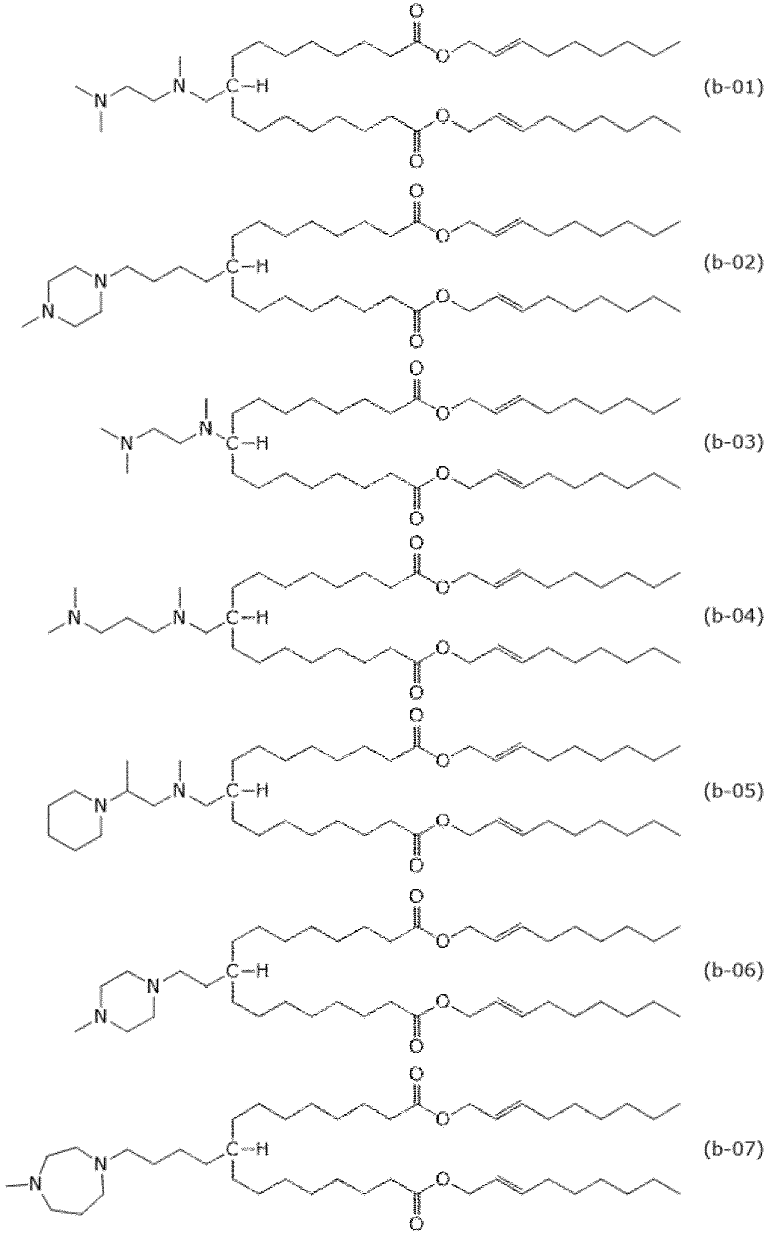

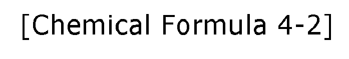

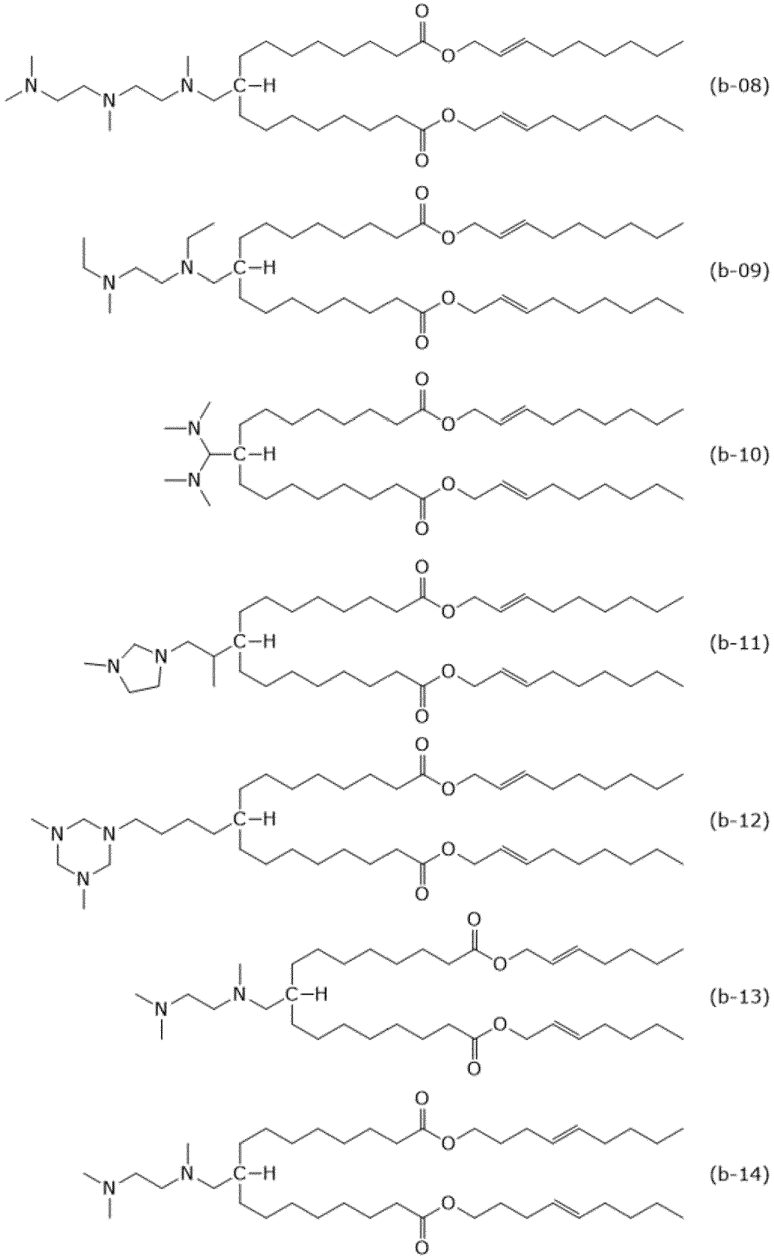

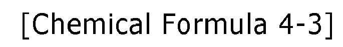

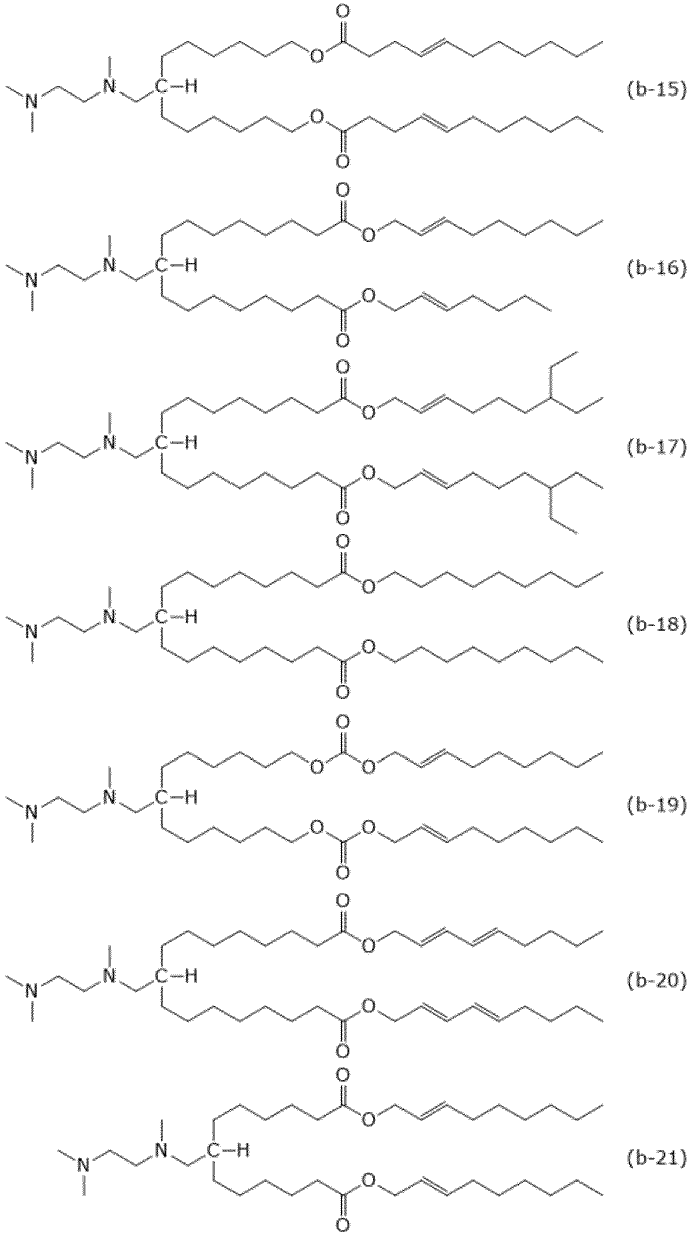

Among them, (b-01) and (b-02) are particularly preferable because they can exhibit excellent properties in a case of being used in liposomes in combination with the compound represented by Formula (a).

The lipid particle according to an embodiment has the compound represented by Formula (a) and, if necessary, the additional lipid compound represented by Formula (b), and can also contain other additional lipid compounds. Such an additional lipid compound can be arbitrarily selected from those generally used for lipid particles.

For example, with respect to the compound of Formula (a), it is also preferable to use dimyristoyl glycerol polyethylene glycol, which is a lipid compound having a structure excluding a maleimide group, in combination with the compound represented by Formula (a).

In addition, it is preferable that an additional lipid compound selected from the group consisting of a cationic lipid, a neutral lipid, a structure-forming lipid, and an aggregation-reducing lipid be further combined. Such a lipid compound is preferably a lipid having excellent biodegradability.

Such a lipid compound effectively acts, for example, to form a liposome membrane. Specific examples thereof include diacyl phosphatidylcholine, diacylphosphatidylethanolamine, ceramide, sphingomyelin, dihydrosphingomyelin, kephalin, and cerebroside. In an embodiment, the lipid forming a membrane used for a lipid particle is appropriately selected in consideration of the size of an intended liposome, the stability of the liposome in a living body, and the like. Among them, diacylphosphatidylcholine and diacylphosphatidylethanolamine are preferable. Here, the length of the hydrocarbon chain of the acyl group contained in the lipid is preferably 10 to 20. The hydrocarbon chain may be a saturated hydrocarbon group or an unsaturated hydrocarbon group.

Various lipids are known as such a lipid that forms a membrane, and preferred examples thereof can include
1,2-dioleoyl-sn-glycero-3-phosphoethanolamine (DOPE),
1,2-distearoyl-sn-glycero-3-phosphoethanolamine (DSPE),
1,2-dipalmitoyl-sn-glycero-3-phosphatidylcholine (DPPC),
1-palmitoyl-2-oleoyl-sn-glycero-3-phosphatidylcholine (POPC),
1,2-di-O-octadecyl-3-trimethylammonium propane (DOTMA),
1,2-dioleoyl-3-dimethylammonium propane (DODAP),
1,2 dimyristoyl-3-dimethylammonium propane (14:0 DAP),
1,2 dipalmitoyl-3- dimethylammonium propane (16:0 DAP),
1,2 distearoyl-3-dimethylammonium propane (18:0 DAP),
N-(4-carboxybenzyl)-N,N-dimethyl-2,3-bis(oleoyloxy)propane (DOBAQ),
1,2-dioleoyl-3-trimethylammonium propane (DOTAP),
1,2 dioleoyl-sn-glycero-3-phosphocholine (DOPC),
1,2-dilinoleoyl-sn-glycero-3-phosphocholine (DLPC),
1,2-dioleoyl-sn-glycero-3-phospho-L-serine (DOPS), and
cholesterol. Among them, DOPE, DOTAP, or cholesterol is more preferable, and a combination of DOPE and cholesterol, a combination of DOTAP and cholesterol, and a combination of DOPE, DOTAP, and cholesterol are particularly preferable. These can exhibit a membrane fusion effect in addition to the function of forming a membrane of a liposome or the like.

The lipid capable of reducing aggregation used in an embodiment exhibits a function including aggregation of particles during preparation of lipid particles. Various lipids are known as such a lipid, and any lipid particle can be selected and used as the lipid particle according to an embodiment. Examples of such a lipid include a polyethylene glycol (PEG) modified lipid, a polyamide oligomer derived from an omega-amino(oligoethylene glycol) alkanoic acid monomer (U.S. Patent No. 6,320,017), and a monosialoganglioside. More specifically, ATTA lipids such as ATTA8-DPSE listed in U.S. Patent No. 6,320,017, and polyethylene glycol lipid conjugates described in U.S. Patent Nos. 5,820,873, 5,534,499, and 5,885,613 can be used.

The PEG-modified lipid can form an anchoring lipid moiety on the surface of the lipid particle when the lipid particle is formed. Examples of such PEG-modified lipids include PEG-modified phosphatidylethanolamines, PEG-modified phosphatidic acids, PEG-ceramide conjugates (for example, C14 PEG-Cer or C20 PEG-Cer, as described in Japanese Patent No. 3920330), PEG-modified dialkylamines, PEG-modified 1,2-diacyloxypropane-3-amines, PEG-modified diacylglycerols (for example, 1,2-dimyristoyl-sn-glycerol-methoxypolyethylene glycol; PEG-DMG), and PEG-modified dialkylglycerols. Among them, PEG-modified diacylglycerols and PEG-modified dialkylglycerols are particularly preferable.

In a case where a bulky modified group such as PEG is bonded to a lipid, the bonding between the modified group and the lipid particle affects the stability of a lipid particle or a liposome. For example, U.S. Patent No. 5,820,873 shows that properties such as the length of an acyl chain, the degree of saturation of an acyl chain, and the size of a sterically hindered head group in a PEG-modified lipid affect the stability of a lipid particle. Therefore, by adjusting these properties, it is possible to obtain a lipid particle according to the purpose. For example, it is possible to cause lipid particles to be lost more quickly by shortening the modified group in the PEG-modified lipid, or it is possible to lengthen the residence time in plasma by lengthening the modified group.

As a result, delivery of lipid particles to target tissues may be improved.

The lipid particle can further comprise other lipids. Such other lipids can be arbitrarily selected from those generally used in lipid particles and used. For example, relatively less toxic lipids can be combined to modulate toxicity. A lipid having a specific structure can also be combined in order to introduce a functional group for binding a ligand to a lipid particle.

Furthermore, in a case where a lipid particle is used as a liposome, sterol, for example, cholesterol can be contained as a lipid for suppressing leakage of the inclusion. Furthermore, a target agonist can be coupled to the lipid particle. As a coupling method in such a case, any conventionally known method can be adopted.

### [Active Agent]

The lipid particle according to an embodiment further comprises an active agent. In an embodiment, the active agent is a substance capable of giving a specific effect to a cell, a tissue, an organ, or a subject. The specific effect may be any of a biological, physiological, or cosmetic effect. By using the lipid particle according to an embodiment, various active agents can be delivered to a target site in a living body. The active agent may be encapsulated within the lipid particle, bound to an outer or inner lipid surface, or disposed within a lipid layer.

A typical example of the active agent is a nucleic acid, and examples thereof include a nucleic acid selected from the group consisting of a plasmid, an oligonucleotide, a polynucleotide, mRNA, small interfering RNA (siRNA), microRNA (miRNA), DNA, an aptamer, and a ribozyme. In addition, an antisense oligonucleotide, an antagonist mir, aDNA, a plasmid, a ribosomal RNA (rRNA), a transfer RNA (tRNA), a small nuclear RNA (snRNA), and the like can also be used. Different kinds of DNA or RNA may be used in combination.

As the miRNA, miRNA in which 17 to 25 nucleotide units are linked can be used. In one more preferred embodiment, the nucleic acid is an oligonucleotide in which 15 to 50 nucleotide units or 20 to 30 nucleotide units are linked. The siRNA can contain, for example, 16 to 30 nucleotide units and have a double-stranded region. In another embodiment, the nucleic acid is an immunostimulatory oligonucleotide, a decoy oligonucleotide, a supermir, an miRNA mimic, or an miRNA inhibitor. The supermir refers to RNA or deoxyribonucleic acid DNA, both of them, or a single-stranded, double-stranded, or partially double-stranded oligomer or polymer of a variant of them, which is an oligomer or a polymer having a nucleotide sequence substantially identical to that of the miRNA and being antisense to a target thereof. The miRNA mimic represents a group of molecules that can be used to mimic the gene silencing ability of one or more miRNAs. Thus, the term "miRNA mimic" refers to a synthetic noncoding RNA capable of entering the RNAi pathway and regulating gene expression (that is, miRNA mimics are not obtained by purification from a source of endogenous miRNAs).

In a case where the nucleic acid is combined with the lipid particle, the form of the nucleic acid is not particularly limited. This nucleic acid can be, for example, single-stranded DNA or RNA, double-stranded DNA or RNA, or a DNA-RNA hybrid. Examples of double-stranded RNA include siRNA. Examples of the single-stranded nucleic acid include an antisense oligonucleotide, a riboenzyme, miRNA, and a triplex-forming oligonucleotide. In a case where the nucleic acid is mRNA, it is preferable that the nucleic acid be modified so as to have degradation resistance. For example, the modification is preferably a known modification that prevents RNA from being degraded by RNase or the like. Such modifications are, for example, the use/introduction of natural modified nucleotides or non-natural nucleotides into RNA, the use/addition of non-natural sequences, or the addition of natural/non-natural CAP structures. Examples of the natural modified nucleotides include pseudouridine, 5-methylcytidine, and 1-methyladenosine. Examples of the non-natural nucleotide include bridged nucleic acid (BNA), locked nucleic acid (LNA), and peptide nucleic acid (PNA). Examples of the non-natural sequence include an artificially produced non-naturally occurring base sequence, and is, for example, a random base sequence, or a hybrid sequence of a natural/non-natural amino acid and a nucleic acid. The non-natural sequence is preferably added, for example, to the end of RNA. The natural CAP structure is, for example, CAPO (m7GpppN), or CAP1 (m7GpppNm). The non-natural CAP structure is, for example, anti-reverse cap analog (ARCA) or LNA-guanosine. The non-natural CAP structure is preferably added, for example, to the 5' end of RNA.

The lipid particle according to an embodiment can further comprise a compound that binds to a nucleic acid in a case of including a nucleic acid. Examples of such a compound include basic proteins or basic peptides, and preferred examples thereof include protamine, a histone, and salts thereof. For example, a histone and a salt thereof have a property of binding to a nucleic acid and folding nucleic acid molecules. In addition, protamine has a property of binding to a nucleic acid and further entrapping a nucleic acid. Therefore, these compounds are effective for encapsulating a nucleic acid in a lipid particle.

In addition, the lipid particle according to an embodiment can further comprise a compound that modulates expression of a nucleic acid in a cell. It is preferable to adjust the expression of the nucleic acid in the cell because an effect of visualization or cell death can be obtained for a cell to which a liposome has been delivered. Examples of such a compound include retinoic acid, cyclic adenosine monophosphate (cAMP), and ascorbic acid.

The lipid particle according to an embodiment may contain a lipoprotein, an apolipoprotein, or the like.

Other therapeutic agents can also be used as an active agent. Specific examples of the therapeutic agent that can be used include a peptide, a polypeptide, a cytokine, a growth factor, an apoptosis factor, a differentiation-inducing factors, a cell surface receptor and a ligand thereof, and a hormone. More specifically, examples of the therapeutic agent include an anti-inflammatory compound, an antidepressant, a stimulant, an analgesic, an antibiotic, a contraceptive, an antipyretic, a vasodilator, an angiogenesis inhibitor, a cytovascular agent, a signal transduction inhibitor, a cardiovascular drug, an oncology drug, a hormone, and a steroid.

When an active agent is combined with the lipid particle, it is preferable that the active agent be introduced into the lipid particle at a higher introduction rate. It is also preferable that cell death due to cytotoxicity dependent on lipid properties be low. In a case where a nucleic acid is introduced using a conventionally known lipid particle, the introduction rate is generally low, and a rate of cell death due to cytotoxicity is also high. On the other hand, in a case where the lipid particle according to the present embodiment is used, the introduction rate of a nucleic acid is high, and cell death can also be reduced. Specifically, in the conventional lipid particle, the introduction rate is about 10%, and the cell death by electroporation is 60% to 70%, whereas in a case of using the lipid particle according to an embodiment, the introduction rate is reduced to 70% or more, and the cell death by electroporation is 30% or less.

The lipid particle according to an embodiment can be formed in any size depending on the purpose. However, in a case where the lipid particle according to an embodiment is used in pharmaceutical applications, the lipid particle is generally a nano-ordered sized particle. Specifically, an average particle size of the lipid particles according to an embodiment is generally 50 nm to 300 nm, and preferably 50 nm to 200 nm. The size of the lipid particle can be adjusted in any manner. For example, a size of the lipid particle can be reduced by sonication. In addition, it is also possible to adjust the size by causing lipid particles to pass through a polycarbonate membrane or a ceramic membrane to be fractionated. In an embodiment, an average particle diameter of the lipid particles can be measured by, for example, a zetasizer using a dynamic light scattering method.

### [Method for Producing Lipid Particle]

The lipid particle according to an embodiment can be produced by any conventionally known method. As a method for producing a lipid particle or a liposome, a Bangham method, an ethanol injection method, an organic solvent extraction method, a surfactant removal method, a freeze-thaw method, and the like are known, and these methods can also be adopted. In addition, for example, a compound represented by Formula (a), further, a lipid forming a membrane, and a lipid capable of reducing aggregation are introduced into an organic solvent such as alcohol, and an aqueous buffer is added, whereby lipid particles can be naturally formed. By combining the active agent with this aqueous buffer solution, it is possible to introduce the active agent into the lipid particles.

### [Application of Lipid Particle]

The lipid particle according to the present embodiment can be used for delivering an active agent to a cell. In particular, delivery of an active agent such as a nucleic acid to a cell is used in all fields such as genetic engineering, production of recombinant proteins, and medical techniques known as gene therapy and cell diagnosis. In one embodiment, there is provided a composition for delivering an active agent to a cell, comprising a lipid particle according to an embodiment and a carrier. In another embodiment, there is provided a lipid particle according to an embodiment for delivering an active agent to a cell. In still another embodiment, there is provided a method of delivering an active agent to a cell, the method comprising bringing a cell into contact with a lipid particle according to an embodiment comprising the active agent (for example, administering the lipid particles to a subject). In still another embodiment, there is provided the use of the lipid particle according to an embodiment for delivering an active agent to a cell. In one embodiment, the cell is a tumor cell. The subject is preferably an animal, more preferably a mammal, and most preferably a human, in need of such treatment. These applications will be specifically described below.

### [Composition]

The lipid particle according to the present embodiment can be used as a composition. For example, there is provided a composition comprising the lipid particle according to the present embodiment and a carrier. Such a composition is also applicable to pharmaceutical applications.

The carrier can be arbitrarily selected from conventionally known carriers and used, and examples thereof include water, saline such as physiological saline, a glycine aqueous solution, and a buffer solution. Furthermore, in addition to these carriers, glycoproteins such as albumin, lipoprotein, apolipoprotein, and globulin may be combined for the purpose of improving stability.

The composition according to an embodiment can be prepared by standard methods. As the carrier, physiological saline is generally used. In the composition including saline or other salt-containing carrier, the carrier is preferably added after formation of the lipid particles. Thus, it is common to replace or dilute the composition with a pharmaceutically acceptable carrier such as saline after combining the lipid particles with an active agent such as a nucleic acid.

The composition according to an embodiment may optionally comprise an adjuvant. For example, for pharmaceutical applications, pharmaceutically acceptable auxiliaries, such as a pH adjuster, a buffering agent, and a tonicity adjuster can be comprised as auxiliaries to bring the pharmaceutical composition closer to a physiological state. Examples of the auxiliary agent having such a function comprise sodium acetate, sodium lactate, sodium chloride, potassium chloride, calcium chloride, and hydroxyethyl piperazine ethanesulfonic acid (HEPES). The composition according to an embodiment can also comprise a lipid protective agent to improve storage stability. Examples of such a protective agent comprise a fatty affinity free radical quencher such as α-tocopherol, which suppresses damage due to free radicals, and a water-soluble chelator such as ferrioxamine, which suppresses damage due to lipid peroxidation.

In addition, the above-mentioned active agent and the like can be added to the composition. The active agent may be the same as or different from the active agent combined with the lipid particle. In addition, a compound that binds to a nucleic acid or a compound that regulates expression of a nucleic acid can be added to the composition.

The concentration of the lipid particles contained in the composition according to an embodiment is not particularly limited, and a content of the lipid particles contained in the composition is generally 0.01 to 30 mass%, and preferably 0.05 to 10 mass%. The concentration of the lipid particles can be appropriately selected according to the purpose.

The composition according to an embodiment can be sterilized by conventionally well-known methods. The sterilized composition can be packaged as a formulation that can be administered as it is, or can be dried and packaged. The dried composition can be formulated for administration by combining with a sterile aqueous solution just prior to administration.

The composition according to an embodiment can also be in kit form. A kit according to an embodiment comprises the above-described lipid particles and an introduction agent for introducing the lipid particles into cells, and a form thereof is arbitrary. Examples thereof include a kit in which a dispersion obtained by dispersing lipid particles not containing an active agent in a carrier and the active agent are stored in individual containers, and a kit in which dried lipid particles, the active agent, and a carrier are stored in individual containers. In addition, the dried lipid particles or lipid particle dispersion and the active agent can be made into individual products so that the user can select each product according to the purpose.

The kit can be combined with a reagent used for nucleic acid introduction.

### [Method for Using Pharmaceutical Composition]

In a case where the lipid particle according to the embodiment is used for pharmaceutical use, the composition can be used for treatment or diagnosis of various diseases in humans or animals. For example, treatment can be performed by delivering a therapeutic agent to a target cell by applying the therapeutic agent as an active agent to be combined with lipid particles.

For example, various nucleic acids can be delivered to and brought into contact with cells to prevent or treat diseases. Such nucleic acids include oligonucleotides, siRNAs, plasmids, antisense, or ribozymes. Lipid compounds according to these embodiments can efficiently and rapidly incorporate these nucleic acids. For example, it has been conventionally difficult to introduce RNA into a lipid particle in a short time and safely, but this can be easily performed by using the lipid compound according to an embodiment.

Also, delivery of an active agent such as a nucleic acid can be performed in vitro, in vivo or ex vivo. As a method of in vivo administration, the pharmaceutical composition preferably adopts parenteral administration, that is, intraarticular administration, intravenous administration, intraperitoneal administration, subcutaneous administration, or intramuscular administration. Intravenous or intraperitoneal administration of the pharmaceutical composition can also be carried out by bolus infusion.

Pharmaceutical preparations according to an embodiment can also be applied directly to a target tissue to bring the pharmaceutical composition into contact with a target tissue. In addition, administration to the meninges or the like by drip infusion or administration by an endoscopic instrument is also possible.

In a certain embodiment, a treatment with the pharmaceutical composition is generally carried out at physiological temperature (about 37°C) for a period of 1 to 24 hours, and preferably for 2 to 8 hours. In in vitro application, a cell to be targeted is not particularly limited. For example, the cell to be targeted may be a vertebrate cell, a non-vertebrate cell, or a plant cell. However, in a preferred embodiment, the cell is an animal cell, more preferably a mammalian cell, and most preferably a human cell. The mammalian cell is preferably a hematopoietic cell, and more preferably selected from the group consisting of PBMC, a T cell, a dendritic cell, and a CD34 positive cell.

### [Examples]

### [Method for Preparing Liposome]

Liposomes encapsulating mRNA (GFP mRNA, TriLink) of GFP gene were prepared by an ethanol injection method. First, a lipid solution was prepared so as to have a lipid composition (unit: % nmol) shown in Table 1. The compounds used are as follows.

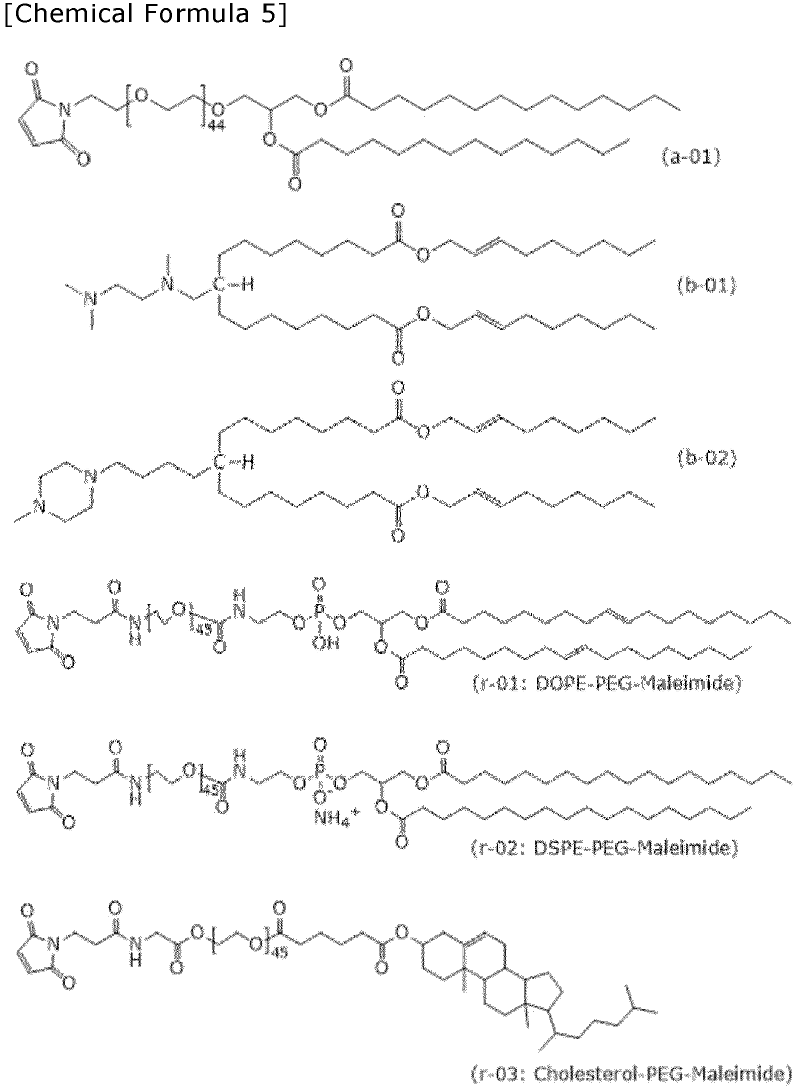

### [Table 1]

**Table 1**

| | (r-01) | (r-02) | (r-03) | (a-01) | (b-01) | (b-02) | DOPE | DOTAP | Cholesterol | PEG |
|---|---|---|---|---|---|---|---|---|---|---|
| Example 101 | 0.0 | 0.0 | 0.0 | 2.0 | 19.6 | 19.6 | 0.0 | 9.8 | 47.0 | 2.0 |
| Example 102 | 0.0 | 0.0 | 0.0 | 0.4 | 19.9 | 19.9 | 0.0 | 10.0 | 47.8 | 2.0 |
| Comparative Example 101 | 0.0 | 0.0 | 0.0 | 0.0 | 20.0 | 20.0 | 0.0 | 10.0 | 48.0 | 2.0 |
| Comparative Example 102 | 2.0 | 0.0 | 0.0 | 0.0 | 19.6 | 19.6 | 0.0 | 9.8 | 47.0 | 2.0 |
| Comparative Example 103 | 0.0 | 2.0 | 0.0 | 0.0 | 19.6 | 19.6 | 0.0 | 9.8 | 47.0 | 2.0 |
| Comparative Example 104 | 0.0 | 0.0 | 2.0 | 0.0 | 19.6 | 19.6 | 0.0 | 9.8 | 47.0 | 2.0 |
| Example 201 | 0.0 | 0.0 | 0.0 | 1.9 | 0.0 | 27.0 | 4.8 | 9.6 | 54.9 | 1.9 |
| Comparative Example 201 | 0.0 | 0.0 | 0.0 | 0.0 | 0.0 | 27.0 | 4.8 | 9.6 | 55.0 | 3.7 |
| Comparative Example 202 | 1.9 | 0.0 | 0.0 | 0.0 | 0.0 | 27.0 | 4.8 | 9.6 | 55.0 | 1.9 |
| Comparative Example 203 | 0.0 | 1.9 | 0.0 | 0.0 | 0.0 | 27.0 | 4.8 | 9.6 | 55.0 | 1.9 |
| Comparative Example 204 | 0.0 | 0.0 | 1.9 | 0.0 | 0.0 | 27.0 | 4.8 | 9.6 | 55.0 | 1.9 |
| Example 301 | 0.0 | 0.0 | 0.0 | 1.0 | 20.0 | 20.0 | 0.0 | 10.0 | 48.0 | 1.0 |
| Comparative Example 301 | 1.0 | 0.0 | 0.0 | 0.0 | 20.0 | 20.0 | 0.0 | 10.0 | 48.0 | 1.0 |
| Comparative Example 302 | 0.0 | 1.0 | 0.0 | 0.0 | 20.0 | 20.0 | 0.0 | 10.0 | 48.0 | 1.0 |
| Comparative Example 303 | 0.0 | 0.0 | 1.0 | 0.0 | 20.0 | 20.0 | 0.0 | 10.0 | 48.0 | 1.0 |

### [(1) Measurement of GFP mRNA Expression Level in Liposome-Administered Cells]

GFP-mRNA-encapsulating liposomes were prepared using the lipid solutions of Comparative Examples 101 to 104 and Example 101. The lipid solution was dispensed into a microtube, and then the GFP mRNA solution was mixed. 10 mM HEPES (pH 7.3) was added to the mixed solution, and buffer exchange and concentration using an ultrafiltration filter (Amicon Ultra) were performed to prepare liposomes.

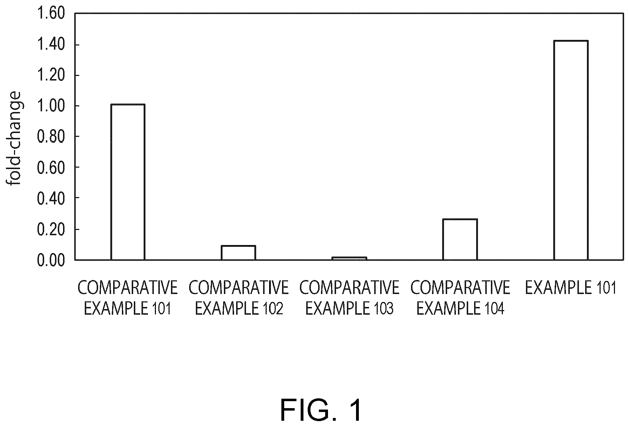

Human peripheral blood mononuclear cells (PBMC) were used as cells. After 2 × 10⁵ cells of PBMCs were seeded on a 96 well culture plate, GFP-mRNA-encapsulating liposomes adjusted to have the above lipid composition were added. After addition of the liposomes, the cells were cultured in an incubator at 37°C and 5% CO₂, and the GFP fluorescence intensity of the cells after 48 hours was measured with a fluorescence-activated cell sorter (SH800S, Sony). The results are shown in Table 2 and Fig. 1. The results are displayed by fold-change, which is a relative ratio obtained by dividing the fluorescence intensity of Example 101 and Comparative Examples 102 to 104 to be measured by the fluorescence intensity of Comparative Example 101 as a control. From Fig. 1, the highest fluorescence intensity (expression of GFP gene) was confirmed in PBMCs to which the GFP-mRNA-encapsulating liposome (Example 101) comprising a lipid compound represented by Formula (a-01) as a lipid compound was administered.

### [Table 2]

**Table 2**

| | fold-change |
|---|---|
| Comparative Example 101 | 1 |
| Comparative Example 102 | 0.08 |
| Comparative Example 103 | 0.02 |
| Comparative Example 104 | 0.27 |
| Example 101 | 1.42 |

### [(2) Measurement of nLuc mRNA Expression Level of Liposome-Administered Cells]

Using the lipid solutions of Example 201 and Comparative Example 202, liposomes encapsulating mRNA of NanoLuc gene (nLuc mRNA) were prepared by an ethanol injection method. The lipid solution was dispensed into a microtube, and then the nLuc mRNA solution was mixed. 10 mM HEPES (pH 7.3) was added to the mixed solution, and buffer exchange and concentration using an ultrafiltration filter (Amicon Ultra) were performed to prepare nLuc-mRNA-encapsulating liposomes.

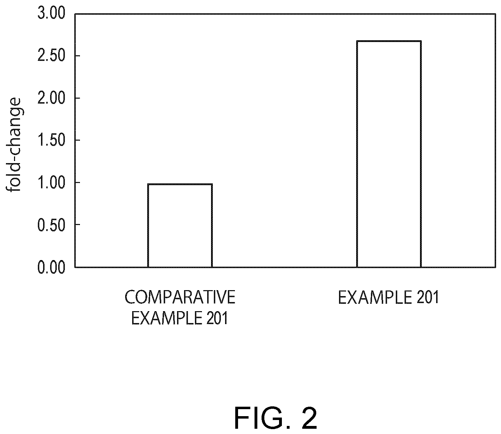

Human T-cell leukemia cells Jurkat were used as cells. After 2 × 10⁵ cells of Jurkat were seeded on a 96 well culture plate, nLuc-mRNA-encapsulating liposomes adjusted to have the above lipid composition were added. After addition of the liposome, the cells were cultured in an incubator at 37°C and 5% CO₂, and nLuc activity of the cells was measured after 48 hours. The enzyme activity of nLuc was measured with a luminometer using NanoGlo Luciferase Assay System (Promega). The results are shown in Table 3 and Fig. 2. The results are displayed by fold-change, which is a relative ratio obtained by dividing the fluorescence intensity of Example 201 to be measured by the fluorescence intensity of Comparative Example 201 as a control. From Fig. 2, the highest fluorescence intensity (expression of nLuc gene) was confirmed in Jurkat to which the nLuc-mRNA-encapsulating liposome comprising a lipid compound represented by Formula (a-01) as a lipid compound was administered.

### [Table 3]

**Table 3**

| | fold-change |
|---|---|
| Comparative Example 201 | 1 |
| Example 201 | 2.65 |

### [(3) Measurement of GFP mRNA Expression Level in Liposome-Administered Cells]

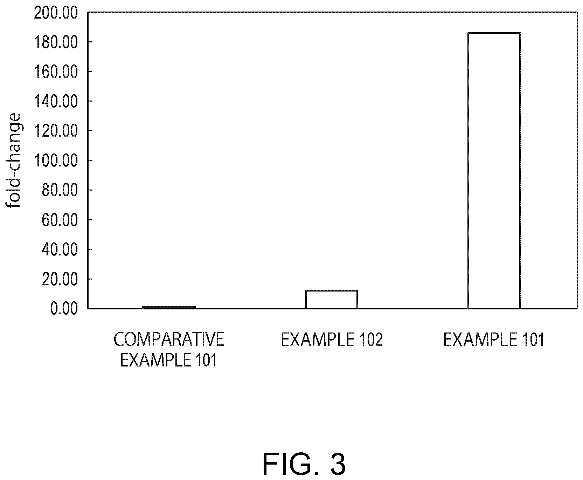

Liposomes encapsulating the lipid solutions of Examples 101 and 102 and Comparative Example 101 were prepared in the same manner as in [(1) Measurement of GFP mRNA Expression Level in Liposome-Administered Cells]. Then, the fluorescence intensity was measured under the same conditions as in [(1) Measurement of GFP mRNA Expression Level in Liposome-Administered Cells] except that a measuring device was changed to a fluorescence-activated cell sorter (FACSverse, BD Biosciences). The results are displayed by fold-change, which is a relative ratio obtained by dividing the fluorescence intensity of Examples 101 and 102 to be measured by the fluorescence intensity of Comparative Example 101 as a control. From Table 4 and Fig. 3, there was a positive correlation between the content of the lipid compound represented by Formula (a-01) in the liposome administered to PBMC and the GFP fluorescence intensity (GFP gene expression level).

### [Table 4]

**Table 4**

| | fold-change |
|---|---|
| Comparative Example 101 | 1 |
| Example 102 | 12.74 |
| Example 101 | 186.21 |

### [(4) Measurement of GFP mRNA Expression Level in Liposome-Administered Cells]

Liposomes encapsulating the lipid solutions of Comparative Examples 201 to 204 and Example 201 were prepared in the same manner as in [(1) Measurement of GFP mRNA Expression Level in Liposome-Administered Cells].

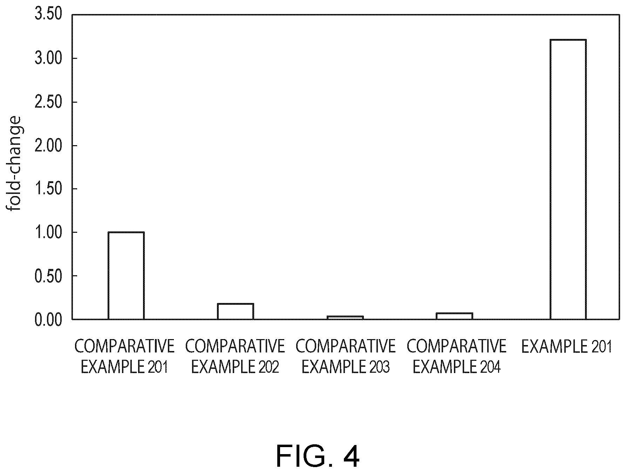

As cells, human mammary adenocarcinoma cells MDA-MB231 were used. After 1 × 10⁵ cells of MDA-MB231 cells were seeded on a 48 well culture plate, GFP-mRNA-encapsulating liposomes adjusted to have the above lipid composition were added. After addition of liposomes, the cells were cultured in an incubator at 37°C and 5% CO₂, and the GFP fluorescence intensity of the cells after 24 hours was measured with a plate reader (infinite F200 PRO, Tecan). The results are shown in Table 5 and Fig. 4. The results are displayed by fold-change, which is a relative ratio obtained by dividing the fluorescence intensity of Example 201 and Comparative Examples 202 to 204 to be measured by the fluorescence intensity of Comparative Example 201 as a control. From Table 5 and Fig. 4, the highest fluorescence intensity (expression of GFP gene) was confirmed in MDA-MB231 to which the GFP-mRNA-encapsulating liposome (Example 201) comprising a lipid compound represented by Formula (a-01) as a lipid compound was administered.

### [Table 5]

**Table 5**

| | fold-change |
|---|---|
| Comparative Example 201 | 1 |
| Comparative Example 202 | 0.17 |
| Comparative Example 203 | 0.04 |
| Comparative Example 204 | 0.7 |
| Example 201 | 3.22 |

### [(5) Measurement of GFP mRNA Expression Level in Liposome-Administered Cells]

Liposomes encapsulating the lipid solutions of Comparative Examples 201 to 204 and Example 201 were prepared in the same manner as in [(1) Measurement of GFP mRNA Expression Level in Liposome-Administered Cells].

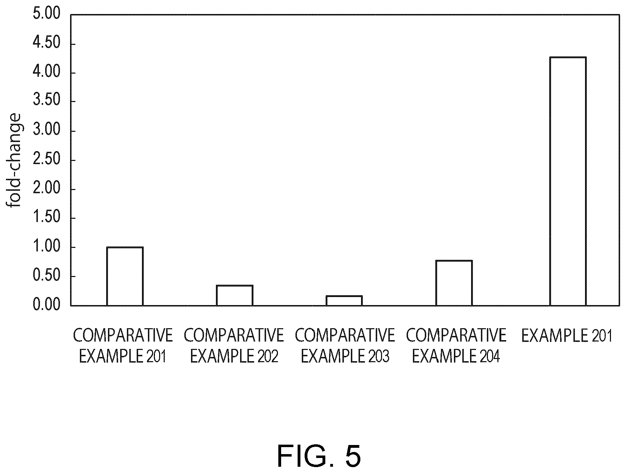

Human T-cell leukemia cells Jurkat were used as cells. After 2 × 10⁵ cells of Jurkat were seeded on a 96 well culture plate, GFP-mRNA-encapsulating liposomes adjusted to have the above lipid composition were added. After addition of liposomes, the cells were cultured in an incubator at 37°C and 5% CO₂, and the GFP fluorescence intensity of the cells after 24 hours was measured with a plate reader (infinite F200 PRO, Tecan). The results are shown in Table 6 and Fig. 5. The results are displayed by fold-change, which is a relative ratio obtained by dividing the fluorescence intensity of Example 201 and Comparative Examples 202 to 204 to be measured by the fluorescence intensity of Comparative Example 201 as a control. From Table 6 and Fig. 5, the highest fluorescence intensity (expression of GFP gene) was confirmed in Jurkat to which the GFP-mRNA-encapsulating liposome (Example 201) comprising a lipid compound represented by Formula (a-01) as a lipid compound was administered.

### [Table 6]

**Table 6**

| | fold-change |
|---|---|
| Comparative Example 201 | 1 |
| Comparative Example 202 | 0.36 |
| Comparative Example 203 | 0.17 |
| Comparative Example 204 | 0.78 |
| Example 201 | 4.29 |

### [(6) Measurement of GFP mRNA Expression Level in Liposome-Administered Cells]

Liposomes encapsulating the lipid solutions of Comparative Examples 101 and 301 to 303 and Example 301 were prepared in the same manner as in [(1) Measurement of GFP mRNA Expression Level in Liposome-Administered Cells].

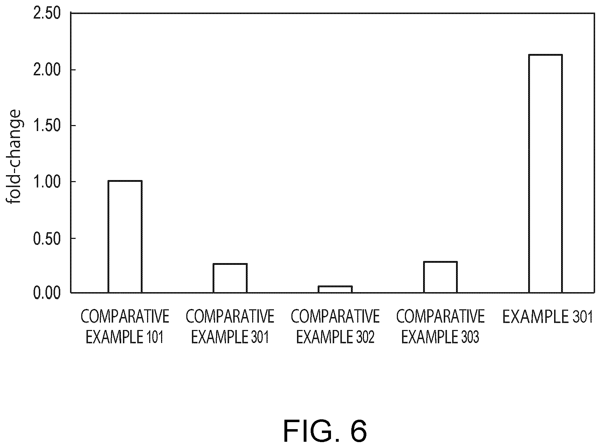

Using the prepared liposomes, the fluorescence intensity was measured by the same method as in [(4) Measurement of GFP mRNA Expression Level in Liposome-Administered Cells]. The results are shown in Table 7 and Fig. 6. The results are displayed by fold-change, which is a relative ratio obtained by dividing the fluorescence intensity of Example 301 and Comparative Examples 301 to 303 to be measured by the fluorescence intensity of Comparative Example 101 as a control. From Table 7 and Fig. 6, the highest fluorescence intensity (expression of GFP gene) was confirmed in MDA-MB231 to which the GFP-mRNA-encapsulating liposome (Example 301) comprising a lipid compound represented by Formula (a-01) as a lipid compound was administered.

### [Table 7]

**Table 7**

| | fold-change |
|---|---|
| Comparative Example 101 | 1 |
| Comparative Example 301 | 0.26 |
| Comparative Example 302 | 0.07 |
| Comparative Example 303 | 0.29 |
| Example 301 | 2.13 |

### [(7) Measurement of GFP mRNA Expression Level in Liposome-Administered Cells]

Liposomes encapsulating the lipid solutions of Comparative Examples 101 and 301 to 303 and Example 301 were prepared in the same manner as in [(1) Measurement of GFP mRNA Expression Level in Liposome-Administered Cells].

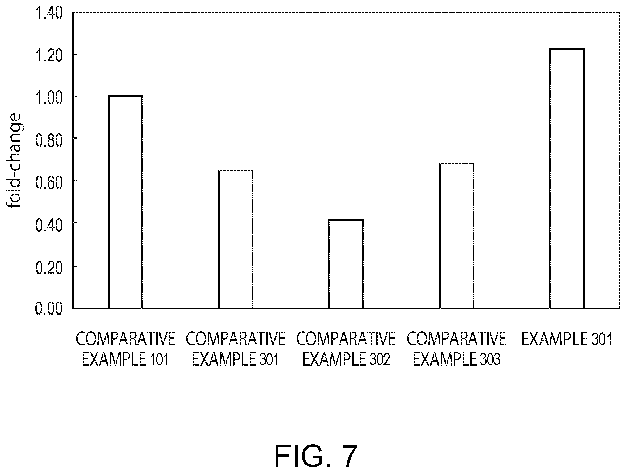

Using the prepared liposomes, the fluorescence intensity was measured by the same method as in [(5) Measurement of GFP mRNA Expression Level in Liposome-Administered Cells]. The results are shown in Table 8 and Fig. 7. The results are displayed by fold-change, which is a relative ratio obtained by dividing the fluorescence intensity of Example 301 and Comparative Examples 301 to 303 to be measured by the fluorescence intensity of Comparative Example 101 as a control. From Table 8 and Fig. 7, the highest fluorescence intensity (expression of GFP gene) was confirmed in Jurkat to which the GFP-mRNA-encapsulating liposome (Example 301) comprising a lipid compound represented by Formula (a-01) as a lipid compound was administered.

### [Table 8]

**Table 8**

| | fold-change |
|---|---|
| Comparative Example 101 | 1 |
| Comparative Example 301 | 0.64 |
| Comparative Example 302 | 0.41 |
| Comparative Example 303 | 0.68 |
| Example 301 | 1.22 |

As described above, some embodiments have been described, but these embodiments have been presented as examples, and are not intended to limit the scope of the invention. These novel embodiments can be implemented in various other forms, and various combinations, omissions, substitutions, changes, and the like can be made without departing from the gist of the invention. These embodiments and modifications thereof are included in the scope and gist of the invention, and are included in the invention described in the claims and the equivalent scope thereof.

## Claims

1. A lipid particle comprising:
a compound represented by the following Formula (a),
Mal-L^{al}-(OCH₂CH₂)ₙₐ-O-L^{a2}R^{a}₂ (a)
wherein, in Formula (a),
Mal is a maleimide group,
L^{a1} is an unsubstituted hydrocarbon group, or a substituted hydrocarbon group containing a linking group selected from the group consisting of -C(=O)-O-, -O-C(=O)-, -O-C(=O)-O-, -S-C(=O)-, -C(=O)-S-, -C(=O)-NH-, and -NH-C(=O)-,
L^{a2} is a trivalent hydrocarbon group that does not contain phosphorus,
each R^{a} is independently a C₁₀-C₃₀ hydrocarbon group containing at least one ester bond at a predetermined position, and
na is a number of 1 or more; and
an active agent.

2. The lipid particle according to claim 1, wherein na is a number from 10 to 100.

3. The lipid particle according to claim 1 or 2, wherein the compound represented by Formula (a) is dimyristoyl glycerol polyethylene glycol maleimide.

4. The lipid particle according to any one of claims 1 to 3, further comprising a compound represented by the following Formula (b),
Q-CHR^{b}₂ (b)
wherein, in Formula (b),
Q is a nitrogen-containing aliphatic group that contains two or more tertiary nitrogen atoms and does not contain oxygen,
each R^{b} is independently a C₁₂-C₂₄ aliphatic group, and at least one R^{b} contains a linking group selected from the group consisting of -C(=O)-O-, -O-C(=O)-, -O-C(=O)-O-, -S-C(=O)-, -C(=O)-S-, -C(=O)-NH-, and - NH-C(=O)- in a main chain or a side chain thereof.

5. The lipid particle according to claim 4,
wherein Q is represented by the following Formula (Q'):
R^{Q1}₂N-(CR^{Q2}₂)_{q1}-NR^{Q1}-(CR^{Q2}₂)_{q2}-*2 (Q')
wherein, in Formula (Q'),
each R^{Q1} is independently an alkyl group,
each R^{Q2} is independently hydrogen or alkyl, or
any two of the R^{Q1} and R^{Q2} groups may form one alkylene group to form a nitrogen-containing alicyclic ring,
q1 is a number of 1 to 5,
q2 is a number of 0 to 5, and
*2 represents a bonding position to -CHR^{b}₂.

6. The lipid particle according to any one of claims 1 to 3, further comprising an additional lipid selected from the group consisting of a cationic lipid, a neutral lipid, a structure-forming lipid, and an aggregation-reducing lipid.

7. The lipid particle according to any one of claims 1 to 6, wherein the active agent is a nucleic acid selected from the group consisting of a plasmid, an oligonucleotide, a polynucleotide, siRNA, microRNA, DNA, mRNA, an aptamer, and a ribozyme.

8. The lipid particle according to claim 7, further comprising a compound that binds to a nucleic acid.

9. The lipid particle according to claim 8, wherein the compound that binds to the nucleic acid is a basic protein or a basic peptide.

10. The lipid particle according to any one of claims 1 to 9, further comprising a compound that modulates expression of a nucleic acid in a cell.

11. A composition comprising: the lipid particle according to any one of claims 1 to 10; and a carrier.

12. A composition for delivering an active agent to a cell, comprising: the lipid particle according to any one of claims 1 to 10; and a carrier.

13. The composition according to claim 12, wherein the cell is a mammalian cell.

14. The composition according to claim 13, wherein the mammalian cell is a hematopoietic cell.

15. The composition according to claim 14, wherein the hematopoietic cell is selected from the group consisting of a PBMC, a T cell, a dendritic cell, and a CD34 positive cell.

16. A method of delivering an active agent to a cell, comprising bringing the lipid particle according to any one of claims 1 to 10 into contact with a cell.

17. The method of delivering an active agent according to claim 16, wherein the cell is a cell of an animal other than a human or is a cell extracted from a living body.

18. The method of delivering an active agent according to claim 16 or 17, wherein the lipid particle further comprises a lipid that forms a membrane and a lipid capable of reducing aggregation.

19. The method of delivering an active agent according to claim 18,
wherein the lipid that forms a membrane is selected from the group consisting of:
1,2-dioleoyl-sn-glycero-3-phosphoethanolamine (DOPE),
1,2-distearoyl-sn-glycero-3-phosphoethanolamine (DSPE),
1,2-dipalmitoyl-sn-glycero-3-phosphatidylcholine (DPPC),
1-palmitoyl-2-oleoyl-sn-glycero-3-phosphatidylcholine (POPC),
1,2-di-O-octadecyl-3-trimethylammonium propane (DOTMA),
1,2-dioleoyl-3-dimethylammonium propane (DODAP),
1,2 dimyristoyl-3-dimethylammonium propane (14:0 DAP),
1,2 dipalmitoyl-3-dimethylammonium propane (16:0 DAP),
1,2 distearoyl-3-dimethylammonium propane (18:0 DAP),
N-(4-carboxybenzyl)-N,N-dimethyl-2,3-bis(oleoyloxy)propane (DOBAQ),
1,2-dioleoyl-3- trimethylammonium propane (DOTAP),
1,2 dioleoyl-sn-glycero-3-phosphocholine (DOPC),
1,2-dilinoleoyl-sn-glycero-3-phosphocholine (DLPC),
1,2-dioleoyl-sn-glycero-3-phospho-L-serine (DOPS), and cholesterol, and
the lipid capable of reducing aggregation is a polyethylene glycol (PEG) modified lipid.

20. The method of delivering an active agent according to any one of claims 16 to 19, wherein the active agent is a nucleic acid selected from the group consisting of a plasmid, an oligonucleotide, a polynucleotide, siRNA, microRNA, DNA, mRNA, an aptamer, and a ribozyme.

21. The method of delivering an active agent according to any one of claims 16 to 19, wherein the active agent comprises a combination of at least one type of DNA and at least one type of RNA.

22. The method of delivering an active agent according to claim 20 or 21, wherein the lipid particle further comprises a compound that binds to a nucleic acid.

23. The method of delivering an active agent according to claim 22, wherein the compound that binds to a nucleic acid is a basic protein or a basic peptide.

24. The method of delivering an active agent according to claim 22, wherein the compound that binds to a nucleic acid is protamine or a histone.

25. The method of delivering an active agent according to claim 22, wherein the lipid particle further comprises a compound that modulates expression of a nucleic acid in a cell.

26. The method of delivering an active agent according to any one of claims 16 to 25, further comprising bringing a carrier into contact with the cell, along with the lipid particle.

27. The method of delivering an active agent according to any one of claims 16 to 26, wherein the cell is a tumor cell.

28. A method of preparing a cell comprising:
performing the method of delivering an active agent according to any one of claims 16 to 27; and
culturing the obtained cell.

29. A kit comprising: the lipid particle according to any one of claims 1 to 10; and an introduction agent that introduces the lipid particle into a cell.

30. The kit according to claim 29, further comprising a substance that improves storage stability of the lipid particle.
